# EUROPEAN PATENT SPECIFICATION

(11) **EP 2 641 229 B1**
(45) Date of publication and mention of the grant of the patent: **05.10.2022**
(21) Application number: 11842307.8
(22) Date of filing: 10.11.2011
(51) Int. Cl.: G16H 20/13

(54) **ALERT NOTIFICATION SERVICE**
ALARMMELDUNGSDIENST
SERVICE DE NOTIFICATION D'ALERTE

(30) Priority: 16.11.2010 US 947736
(43) Date of publication of application: 25.09.2013
(73) Proprietor: CareFusion 303, Inc., San Diego, CA 92130 (US)
(72) Inventor: NUDELMAN, Anthony, Poway, California 92064 (US); SHELTON, Stephen, Encinitas, California 92024 (US); UTECH, Thomas, San Diego, California 92103 (US); GODLEWSKI, Peter, San Clemente, California 92121 (US); JING, Emily, San Diego, California 92129 (US); SCHMIDT, Charlie, San Diego, California 92108 (US)
(74) Representative: Epping - Hermann - Fischer
(86) International application number: PCT/US2011/060274
(87) International publication number: WO 2012/067950

(56) References cited:
- US-A- 5 408 443
- US-A1- 2001 056 358
- US-A1- 2003 236 683
- US-A1- 2006 149 416
- US-A1- 2007 219 825
- US-A1- 2009 259 486
- US-B2- 7 657 443

## Description

### BACKGROUND

### Field

The present disclosure generally relates to medication dispensing, and particularly to providing alerts related to a patient receiving the dispensed medication.

### Description of the Related Art

It is well known that a physician prescribes medications to a patient in order to improve the health of the patient. In many such circumstances, the medications are prescribed for the patient after the physician reviews critical lab values (e.g., related to infections, resistances, susceptibilities) associated with the patient's health. For example, if a lab value indicates the patient's cholesterol level is too high, the physician may prescribe a cholesterol lowering medication. It is also well known in the medical community, and in particular, in hospitals, to provide centrally located medication and supply dispensing stations, such as wall cabinets, manually secured patient cassette drawers, and automated dispensing machines (ADM) to facilitate the distribution of these medications to patients, such as by hospital nurses.

A hospital nurse is usually responsible for dispensing medications several times a day to a patient for whom he or she administers care. The medications are commonly retrieved for dispensing by the hospital nurse from the ADM after the hospital nurse provides proper authentication information (e.g., a user identification). During dispensing, a nurse is often unaware of the critical lab values relating to his or her patient obtained after a physician performs his or her daily round of patient examinations. If a lab value returns with an indication that treatment with a medication be discontinued, the nurse has few or no opportunities to learn of the treatment change until the next day. If continued treatment with the medication is dangerous to the health of the patient, the delay in providing information on the treatment change to the nurse will result in avoidable and unintended harm to the patient. For example, if the patient's most recent lab values indicate his cholesterol level is dangerously low, it would be harmful to continue to provide cholesterol lowering medications to the patient.

US 2003/236683 A1 relates to a closed loop medication use system and method containing computer hardware and software connected to facilitate communication and perform tasks to assist medical professionals in patient care.

US 2006/149416 A1 relates to a system, software, and related methods for providing pharmaceutical services and inventory control.

US 7 657 443 B2 relates generally to systems and methods for managing patient care in a health care facility, and more particularly, to systems and methods for assessing the severity of averted medication errors.

US 2001/056358 A1 relates to the prevention of errors in medication administration, and in particular, to a method, apparatus, system, and article of manufacture for providing medication administration warnings and comments.

US 2007/219825 A1 relates to methods for delivering a drug to a patient. More particularly, the methods restrict access to the drug to only those patients for which the risk that they will experience an adverse side effect is acceptable. This restricted drug distribution method enables healthcare providers to manage and reduce side effects associated with exposure to a contraindicated condition.

US 2009/259486 A1 relates to a patient centric medication dispensing device configured to close the loop between a patient, a treating physician and a pharmacy by monitoring the medication intake of the patient and reporting information to the physician and/or the pharmacy or pharmaceutical company. The dispensing device is further configured to calculate adverse drug effects that may result if a patient takes a requested medication, based on medication that has already been dispensed and medication that is scheduled to be taken. The dispensing device is also configured to provide the patient with educational information based on the situation the patient has encountered, such as missing a scheduled dosage or suffering from side-effects.

US 5 408 443 A relates to a medication-dispensing system including a prescribing data entry station for use by a physician to store prescription information in a portable prescribing module, a dispensing data entry station for use by a pharmacist to store dispensing information in a portable dispensing data storage unit, and a medication dispenser responsive to information stored in the portable prescribing module to describe use of medication in the dispenser in accordance with a regimen prescribed by the physician and to the dispensing data storage unit to control dispensing of the medication.

### SUMMARY

There is a need for a system and method that provides updated information on treatment changes to a nurse at the time the nurse is obtaining and dispensing medications to a patient. The disclosed system, according to certain embodiments, uses an ADM to display up-to-date information on treatment changes specific to a patient and specific to the patient's medications, based on the most recently available critical lab values, when a nurse attempts to dispense medications from the ADM.
The invention is defined by the claims. The examples, aspects and embodiments, if any, disclosed in the following description that do not fall within the scope of the claims are for reference only, and are to be interpreted as examples useful for understanding various embodiments of the invention.

According to certain embodiments of the present disclosure, a system for providing medication dispense alert notifications is provided. The system includes a communications module configured to receive, from a client, a request for information regarding a medication dispense for a patient, and to obtain clinical information associated with the patient based on the request. The system also includes a processor configured to determine, based on the clinical information, whether a response to be provided to the client will include an alert regarding the medication dispense. The communications module is further configured to provide, to the client, the response to the request.

According to certain embodiments of the present disclosure, a method for providing medication dispense alert notifications is provided. The method includes receiving, from a client, a request for information regarding a medication dispense for apatient, and obtaining clinical information associated with the patient based on the request. The method also includes determining, using a processor and based on the clinical information, whether a response to be provided to the client will include an alert regarding the medication dispense, and providing, to the client, the response to the request.

According to certain embodiments of the present disclosure, a machine-readable medium comprising machine-readable instructions for causing a processor to execute a method for providing medication dispense alert notifications is provided. The method includes receiving, from a client, a request for information regarding a medication dispense for a patient, and obtaining clinical information associated with the patient based on the request. The method also includes determining, using a processor and based on the clinical information, whether a response to be provided to the client will include an alert regarding the medication dispense, and providing, to the client, the response to the request.

According to certain embodiments of the present disclosure, a system for providing medication dispense alert notifications is provided. The system includes a communications module configured to receive a selection of a patient for a medication dispense, to provide, to a server, a request for information regarding the medication dispense for the patient, and to receive, from the server, a response to the request. The system also includes a processor configured to instruct a display device to display an alert window if the response includes an alert regarding the medication dispense.

According to certain embodiments of the present disclosure, a system for dispensing medications to a patient is provided. The system includes an input device configured to receive, from a user, a request to dispense a medication for a patient, and a communications module configured to provide, to a server, a request for information regarding the medication dispense for the patient, and to receive, from the server, a response to the request. The response to the request includes an indicator that identifies the medication as potentially harmful to the patient based on clinical information for the patient. The system also includes a processor configured to instruct a display device to display, to the user, an alert window comprising the indicator that identifies the medication as potentially harmful to the patient based on clinical information for the patient.

According to certain embodiments of the present disclosure, a method for providing medication dispense alert notifications is provided. The method includes receiving a selection of a patient for a medication dispense, and providing, to a server, a request for information regarding the medication dispense for the patient. The method also includes receiving, from the server, a response to the request, and displaying, using a processor, an alert window if the response includes an alert regarding the medication dispense.

According to certain embodiments of the present disclosure, a machine-readable medium comprising machine-readable instructions for causing a processor to execute a method for providing medication dispense alert notifications is provided. The method includes receiving a selection of a patient for a medication dispense, and providing, to a server, a request for information regarding the medication dispense for the patient. The method also includes receiving, from the server, a response to the request, and displaying, using a processor, an alert window if the response includes an alert regarding the medication dispense.

### BRIEF DESCRIPTION OF THE DRAWINGS

The accompanying drawings, which are included to provide further understanding and are incorporated in and constitute a part of this specification, illustrate disclosed embodiments and together with the description serve to explain the principles of the disclosed embodiments. In the drawings:
FIG. 1 illustrates an exemplary architecture for a medication dispensing alert notification service according to certain embodiments.
FIG. 2 is an exemplary process for providing medication dispensing alerts using the architecture of FIG. 1.
FIGS. 3A-3G are exemplary screenshots that illustrate various steps of the process of FIG. 2 and features of the service of FIG. 1.
FIG. 4 is a block diagram illustrating an example of a computer system with which the ADM, console, or the server illustrated in FIG. 1 can be implemented.

### DETAILED DESCRIPTION

In the following detailed description, numerous specific details are set forth to provide a full understanding of the present disclosure. It will be obvious, however, to one ordinarily skilled in the art that the embodiments of the present disclosure may be practiced without some of these specific details. In other instances, well-known structures and techniques have not been shown in detail not to obscure the disclosure.

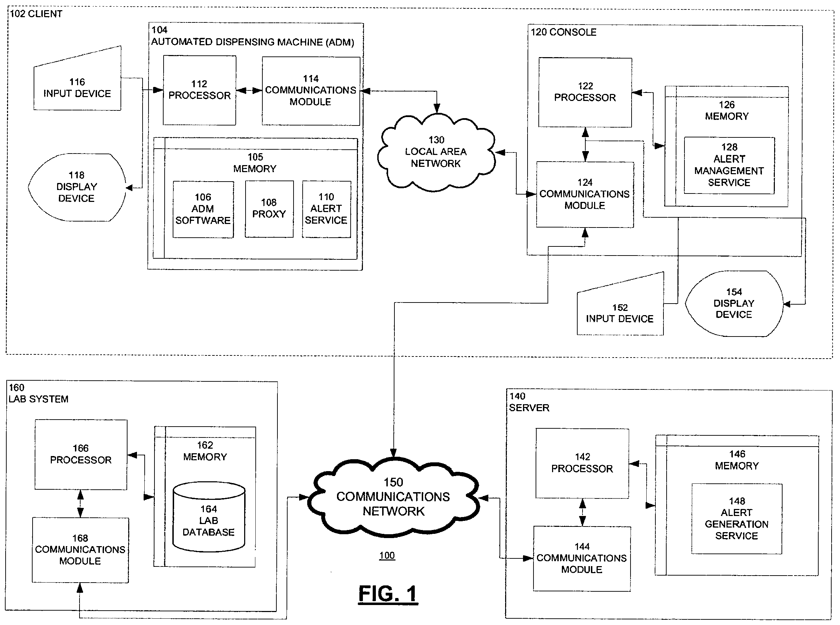

FIG. 1 illustrates an exemplary architecture 100 for a medication dispensing alert notification service according to certain embodiments. The architecture 100 includes a client 102 and a server 140. The client 102 includes an automated dispensing machine 104 (ADM) and a console 120. The ADM 104 is connected, wired or wirelessly, to the console 120 over a local area network 130, and the console 120 connects the client 102 to the server 140 over a communications network 150. The local area network 130 can be, for example, a private communications network, and the communications network 150 can be, for example, another local area network, or a wide area network. Exemplary communications networks 150 include.the Internet or a private communications network.

The server 140 includes a processor 142, a communications module 144, and a memory 146 that includes an alert generation service 148. The server 140 is configured for communication over the communications network 150 (e.g., with console 120) using a communications module 144. The communications module 144 can be, for example, a modem or Ethernet card. The communications module 144 is configured to receive, from the client 102, a request for information regarding a medication dispense for a patient. The communications module 144 is also configured to obtain clinical information for the patient, such as from a lab database 164 stored in a memory 162 of a lab system 160 connected to the server 140 over the communications network 150. Exemplary lab systems (or software for such systems) that provide clinical information configured for use with the disclosed system include, but are not limited to, MedMined by CareFusion, Theradoc by Hospira, Sentri7 by Pharmacy OneSource, CareExpert by Thomson-Reuters, Safety Surveillor by Premier, Vigilanz by Quantros, and Vecna by Advisor Board. In certain embodiments, the clinical information includes, for example, lab values, lab results, and vital signs. In certain embodiments, the clinical information includes an indication as to whether it includes critical information, such as out of range lab values, abnormal lab results, or abnormal vital signs. In certain embodiments, the clinical information is obtained after receipt of the request from the client, for example, to ensure that the clinical information is up to date. Similarly, in certain embodiments, the clinical information is updated based on a most recently recorded physician round.

The processor 142 of the server 140 is configured to execute instructions, such as instructions physically coded into the processor 142, instructions received from software in memory 146, or a combination of both. For example, the processor 142 of the server 120 is configured to determine, using alert generation service 148, and based on clinical information received by the communications module 144 from the client 102, whether a response to be provided to the client 102 will include an alert regarding the medication dispense. The communications module 144 is further configured to provide, to the client 102, the response to the request. The response to the request is generated by the alert generation service 148. In certain embodiments, the alert is included in the response if the clinical information comprises a critical indicator. For example, if the clinical information indicates that a lab value is critically out of range, then an alert will be included in the response that provides such an indication of the lab value. Thus, in certain embodiments, an alert may include at least one of a lab value, a time value, a medication, a query, an instruction, and an acknowledgement. In certain embodiments, the response is provided by the alert generation service 148 of the server 140 to the alert management service 128 of the console 120.

The client 102 includes at least two devices, the ADM 104 and the console 120. The ADM 104 and the console 120 are configured for communication over the local area network 130 using their respective communications modules, 114 and 124. The communications modules 114 and 124 can be, for example, modems or Ethernet cards.

The console 120 includes a processor, a communications module 124, an input device 152, a display device 154, and a memory 126 that includes an alert management service 128. The communications module 124 is configured to receive the request for information from the ADM 104, provide the request to the server 140, receive the response to the request for information regarding a medication dispense for a patient from the server 140, and provide the response to the ADM 104. Although the communications module 124 of the console 120 is illustrated as connected to a single ADM 104, the communications module 124 is configured to connect to a plurality of ADMs 104, such that the communications module 124 is configured to receive a plurality of requests for information from a plurality of ADMs 104, provide the plurality of requests to the server 140, receive responses to the plurality of requests from the server 140, and provide the plurality of responses to the appropriate ADM 104 from the plurality of ADMs 104. Thus, all of the requests are made to the server from the console 120, which allows the alert management service 128 of the console 120 to keep a log of the requests and have access to service status information of the connected ADMs 104. The alert management service 128 is thus configured for centralized management of alert related operations where a plurality of ADMs exist.

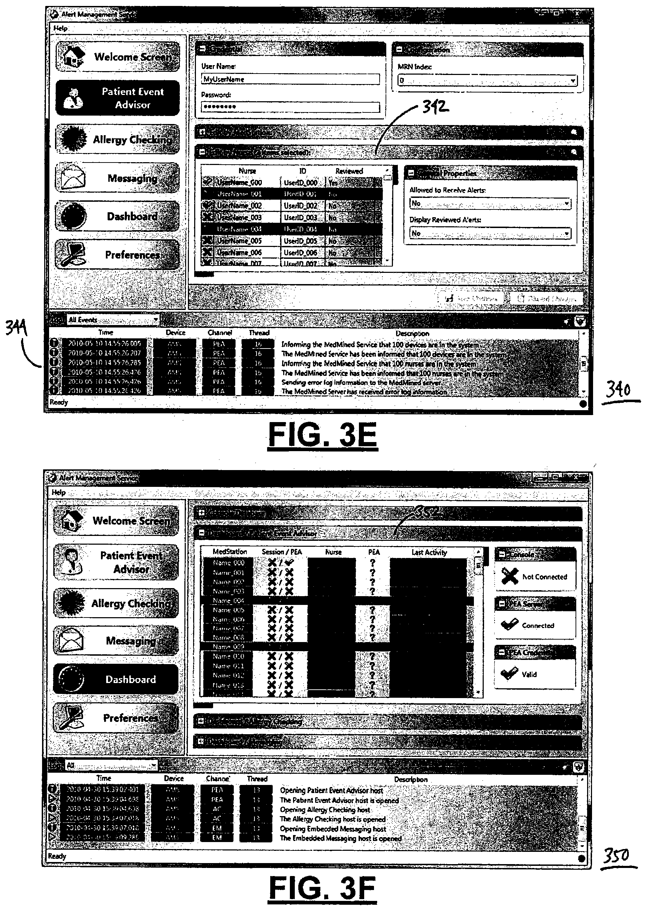

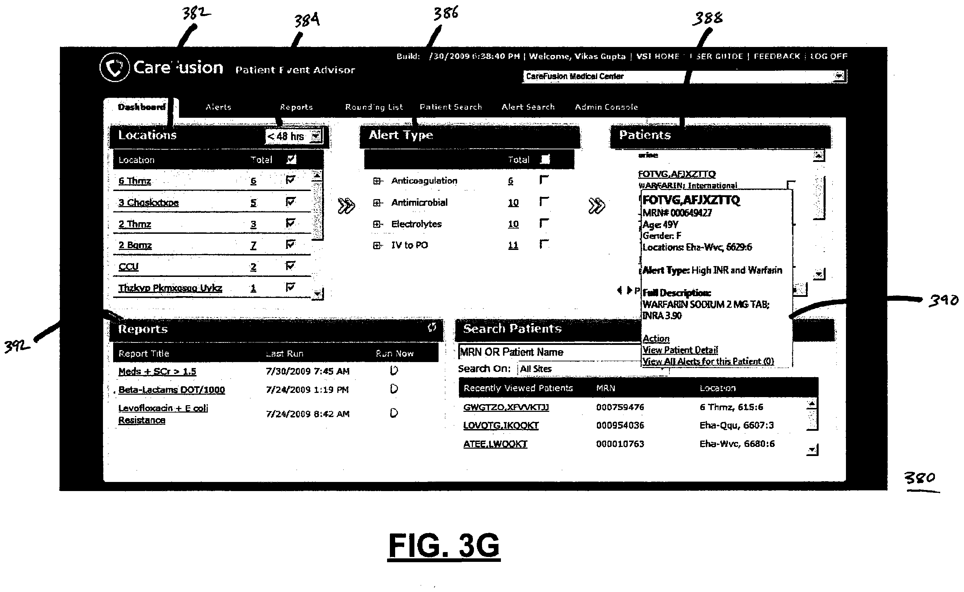

The processor 122 of the console 120 is configured to execute instructions, such as instructions physically coded into the processor 122, instructions received from software in memory 126, or a combination of both. For example, the processor 122 of the console 120 is configured to provide a user interface for managing an alert service, as illustrated in FIGS. 3E and 3F, described in further detail below, as well as a patient event advisor user interface, as illustrated in FIG. 3G, described in further detail below. Continuing with FIG. 1, the user interfaces can be displayed on display device 154 and receive input via input device 152. In certain embodiments, either of the alert service user interface and the patient event advisor user interface require authentication, such as a username and password, to ensure that access to the user interfaces are restricted to authorized users.

The ADM 104 includes a processor 112, communications module 114, an input device 116, a display device 118, and a memory 105 that includes ADM software 106, a proxy 108, and an alert service 110. In certain embodiments, the ADM software 106 and the alert service 110 are pre-existing software loaded in the memory 105 of the ADM 104. Thus, by installation of the alert service 110 on an ADM 104 having the appropriate ADM software 106 and alert service 110, the ADM 104 is compatible with the disclosed systems. In certain embodiments, the alert service 110 functions as a standalone executable running in the background of an operating system. Exemplary ADMs include the Pyxis line of MedStations^{®} by CareFusion. Other ADMs may also be used if they are configured as described above. In certain embodiments, a point-of-care dispensing device may be used instead of an ADM.

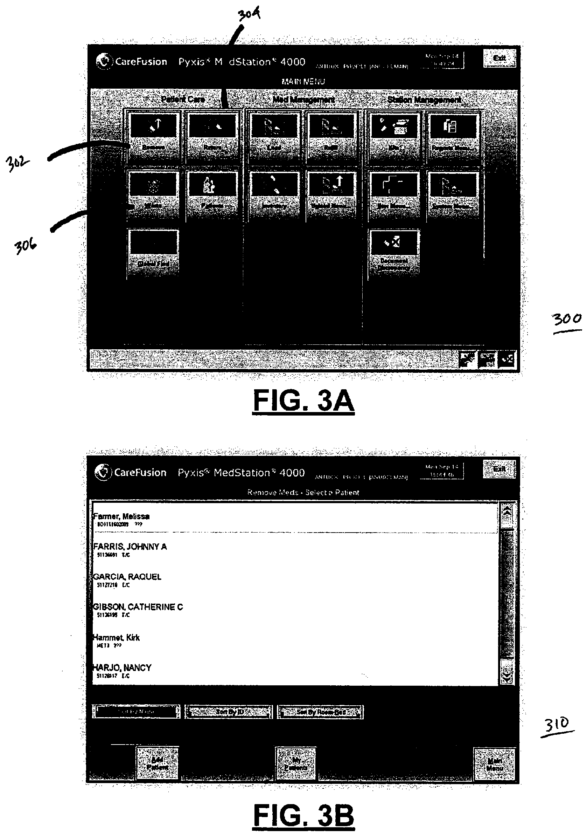

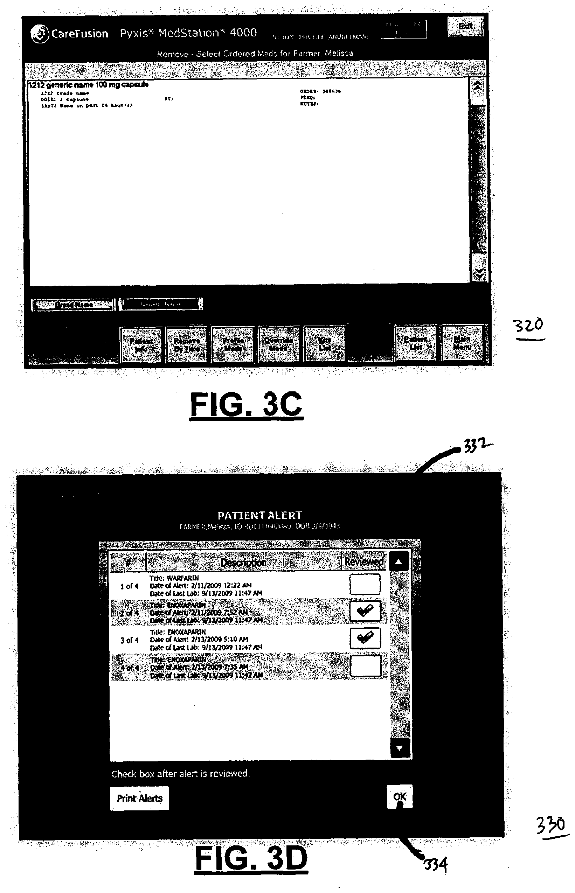

The communications module 114 of the ADM 104 is configured to receive a selection (e.g., from a user using input device 116, as illustrated in FIGS. 3A-3C, described in further detail below) of a patient for a medication dispense that triggered the request for information regarding the medication dispense for the patient, and provide the request for information to the console 120. The communications module 114 is also configured to receive the response to the request for information regarding a medication dispense for a patient from the console 120. The processor 122 of the console 120 is configured to execute instructions, such as instructions physically coded into the processor 122, instructions received from software in memory 126, or a combination of both. For example, the processor 122 of the console 120 is configured by proxy 108 to instruct the alert service 110 to process the response to the request for information, and is configured by proxy 108 to instruct the display device 118 to display an alert window if the response to the request for information includes an alert regarding the medication dispense, as illustrated in FIG. 3D, described in further detail below. The displayed alert can include information such as, but not limited to, a title, a lab value (either in a normal range or abnormal range), a time value, a medication, a query, an instruction (e.g., to not dispense the medication), access to a reference database (e.g., Lexi-Comp), and an acknowledgement (e.g., that the user has reviewed the displayed alert). For example, the alert may display a title of the alert, date of the alert, and a date of the last laboratory results associated with the alert. In certain embodiments, if either the local area network 130 or the communications network 150 is unavailable, the processor 122 of the console 120 is configured by proxy 108 to display on the display device 118 an alert that the local area network 130 or the communications network 150 is unavailable, thereby providing an indication to a user (e.g., a nurse) attempting to dispense medications from the ADM that alerts regarding the medication dispense are not available.

In certain embodiments, the transmission of the request for information regarding the medication dispense for the patient and the response to the request are transmitted over various data channel types and/or formats, such as, but not limited to, a Transmission Control Protocl (TCP)/Internet Protocol (IP) request/response format, a shared data channel, a separate data channel, in polling format, via File Transfer Protocol (FTP), or Simple Object Access Protocol (SOAP) format.

In certain embodiments, the console 120 may be removed from the architecture 100 by providing the alert management service 128 in the memory 105 of the ADM 104 and connecting the ADM 104 to the server 140 over the communications network 150. Alternatively, the console 120 may be removed from the architecture 100 by configuring the alert service 110 (via proxy 108) in the memory 105 of the ADM 104 to receive the response to the request for information directly from the server 140 over the communications network 150.

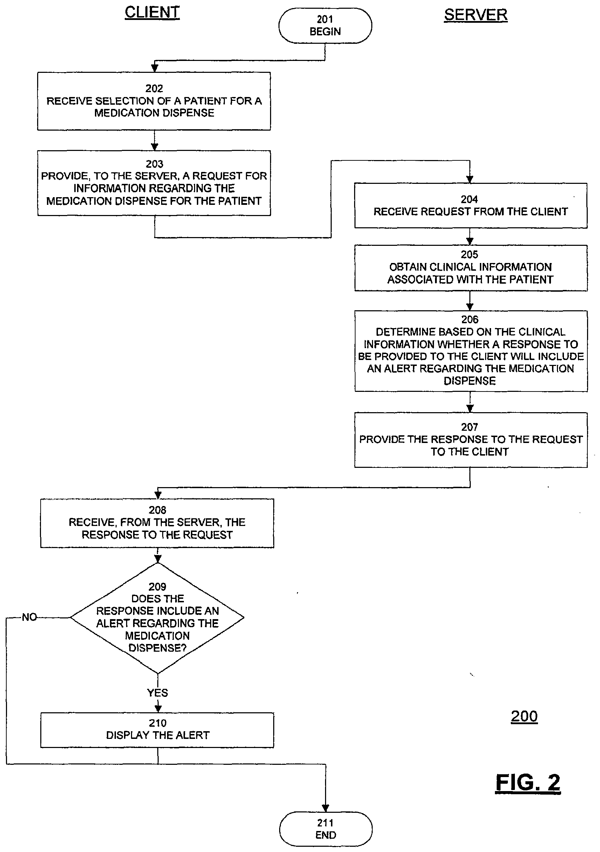

FIG. 2 is an exemplary process 200 for providing medication dispensing alerts using the architecture 100 of FIG. 1. The process 200 proceeds from beginning step 201 to step 202 in which a selection of a patient for a medication dispense is received. In step 203, a request for information regarding the medication dispense for the patient is provided to the server 140. In step 204, the server 140 receives the request from the client 102, and in step 205 clinical information (e.g., vital signs, lab results) associated with the patient is obtained. Next, in step 206, it is determined, based on the clinical information, whether a response to be provided to the client 102 will include an alert regarding the medication dispense. In step 207, the response to the request is provided to the client 102. Returning to the client 102 side, in step 208, the response to the request is received from the server 140. In decision step 208, if the response includes an alert regarding the medication dispense, then the alert is displayed by the display device 118 of the client 102 (e.g., ADM 104) in step 210 and the process 200 ends in step 211. If, however, in decision step 208, the response does not include an alert regarding the medication dispense, then the process 200 ends in step 211. In certain embodiments, if the alert is received by the ADM 104 after the user has finished dispensing for the selected patient, then the alert is not displayed.

Having set forth in FIG. 2 a process 200 for providing medication dispensing alerts using the architecture 100 of FIG. 1, an example will now be presented using the process 200 of FIG. 2, an authorized nurse as the user, and the exemplary screenshots of FIGS. 3A-3D. The process 200 is initiated, for example, when the nurse approaches an ADM to retrieve medications to dispense to a patient. The nurse logs in to the ADM 104, if necessary, and the exemplary screenshot 300 of FIG. 3A is displayed to her on the display device 118 of the ADM 104. The process 200 then proceeds from beginning step 201 to step 202 in which the nurse makes appropriate selections of an action and a patient from the display device 118 via an input device 116. The screenshot 300 from the display device 118 includes various patient care functions, including removing a medication 302, returning a medication 304, or wasting a medication 306 that may be selected by the nurse using the input device 116. As her first selection in step 202, the nurse chooses to remove a medication 302, and the display then prompts the nurse with a patient list to select the patient for whom she is removing the medication, as illustrated in the exemplary screenshot 310 of FIG. 3B. After the nurse selects the patient, in step 203, a request for information regarding the medication dispense for the selected patient is provided to the server 140. In certain embodiments, the request is a non-blocking function call. For example, it allows the nurse to continue to use the input device 116 to interact with the ADM 104, which in response to the selection of the patient displays a list of medications associated with the patient, as illustrated in the exemplary screenshot of FIG. 3C. In step 204, the server 140 receives the request from the client 102, and in step 205 clinical information (e.g., vital signs, lab results) associated with the patient is obtained. Next, in step 206, it is determined, based on the clinical information, that a response to be provided to the client 102 will include an alert regarding the medication dispense because the clinical information includes critical values that will be impacted by certain medication associated with the patient, such as Warfarin and Enoxaparin. Specifically, the patient's latest critical lab values indicated that the patient's bleeding time is out of range (i.e., the International Normalized Ratio for the patient was a value between 2.0 to 3.0, and the Factor Xa value was out of range), therefore the patient should not be administered Warfarin and Enoxaparin because it would aggravate the condition. In step 207, the response to the request is provided to the client 102.

Returning to the client 102 side, in step 208, the response to the request is received from the server 140, and in decision step 208, because the response includes an alert regarding the medication dispense, and the nurse is still viewing the selected patient on the display device 118, the alert is displayed on the display device 118, as illustrated in the exemplary screenshot 330 of FIG. 3D. As illustrated in the screenshot 330 of the displayed alert, the nurse's usual workflow process on the ADM 104 has been interrupted in order to display the patient alert window 332. The nurse, being unaware at the time of dispensing that the patient's bleeding time is out of range, is instructed not to administer Warfarin and Enoxaparin to the patient, thereby avoiding harming the patient, even possibly saving the patient's life. The remaining screen area beyond the patient alert window 332 has been darkened and the nurse is prevented from interacting with the ADM software 106 until the alert window 332 is closed, ensuring that the nurse acknowledges the alert. The alert window 332 provides the nurse with the option to review each of the four alerts for the medications displayed in the alert window 332. The nurse selects the acknowledgement button 334 and resumes the workflow process on the ADM 104. In certain embodiments, if the nurse does not interact with the alert window 332, an optional timeout window (not illustrated) is displayed that provides a countdown to logging off the nurse from the ADM 104 in order to prevent unauthorized access or interaction. A response to the timeout window cancels the log off procedure. The process 200 ends in step 211. In certain embodiments, if an alert is marked as reviewed by the nurse, the alert is no longer displayed the next time the the nurse selects the same patient and same medication, thereby avoiding notification desensitization (e.g., the nurse not paying attention to alerts because they appear to often and/or are irrelevant because they have been previously viewed).

FIGS. 3E and 3F are exemplary screenshots 340 and 350 generated by the alert management service 128 of the console 120 and displayed on the display device 154 of the console 120. In certain embodiments, the alert management service 128 user interface is accessible after user authentication. The alert management service 128 is configured to allow a user to view previously reviewed alerts that have been displayed by the ADM 104. For example, the alert management service user interface 340 illustrated in the screenshot of FIG. 3E includes a list 342 of previously displayed alerts and whether they were reviewed. The management service user interface 340 also includes a log 344 of alert actions. As illustrated in the alert management service user interface 340 screenshot of FIG. 3F, the user interface 340 can also include a list 352 of activities taken by a nurse sorted by ADM 104. The alert management service 128 is also configured to allow a user (e.g., an administrator) to configure which alerts are displayed on the ADM 104 (e.g., if the ADM 104 is located in an Emergency Room department, then the ADM 104 may be configured to receive and display only essential alerts). This provides a centralized user interface to configure any ADM 104 connected to the console 104, thus obviating the need to configure each ADM 104 independently. For example, authentication information for healthcare providers (e.g., nurses) authorized to use any ADM 104 connected to the console 104 can be assigned using the alert management service 128 of the console 120.

FIG. 3G is an exemplary screenshot of a patient event advisor user interface 380 displayed on the display device 154 of the console 120. In certain embodiments, the patient event advisor user interface 380 is accessible after user authentication. The patient event advisor user interface 380 provides a user with information to monitor, in real-time, patient (e.g., hospital inpatient) information for potential adverse clinical events to medications. The patient event advisor user interface 380 assists users (e.g., clinical pharmacists) in identifying patients of high interest and tracks their therapy for changing needs. The patient event advisor user interface 380 supports hospital performance initiatives with quality metrics reporting, and provides workflow documentation for clinical interventions. The patient event advisor user interface 380 provides information on alerts organized by location 382 (filtered by time 384), by alert type 386, and by patient 388. A pop-out window 390 provides key patient information for the listed patients 388. The patient event advisor user interface 380 also provides reports, such as in response to ad hoc patient queries and custom report requests.

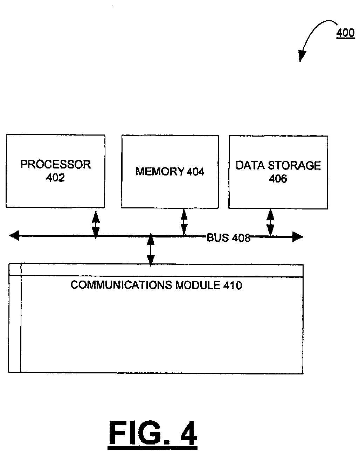

FIG. 4 is a block diagram illustrating an example of a computer system with which the ADM 104, console 120, or the server 140 illustrated in FIG. 1 can be implemented. In certain embodiments, the computer system 400 may be implemented using software, hardware, or a combination of both, either in a dedicated server, or integrated into another entity, or distributed across multiple entities.

Computer system 400 (e.g., ADM 104, console 120, or server 140 from FIG. 1) includes a bus 408 or other communication mechanism for communicating information, and a processor 402 (e.g., processor 112, 122, or 142 from FIG. 1) coupled with bus 408 for processing information. By way of example, the computer system 400 may be implemented with one or more processors 402. Processor 402 may be a general-purpose microprocessor, a microcontroller, a Digital Signal Processor (DSP), an Application Specific Integrated Circuit (ASIC), a Field Programmable Gate Array (FPGA), a Programmable Logic Device (PLD), a controller, a state machine, gated logic, discrete hardware components, or any other suitable entity that can perform calculations or other manipulations of information. Computer system 400 also includes a memory 404 (e.g., memory 105, 126, or 146 from FIG. 1), such as a Random Access Memory (RAM), a flash memory, a Read Only Memory (ROM), a Programmable Read-Only Memory (PROM), an Erasable PROM (EPROM), registers, a hard disk, a removable disk, a CD-ROM, a DVD, or any other suitable storage device, coupled to bus 408 for storing information and instructions to be executed by processor 402. The instructions may be implemented according to any method well known to those of skill in the art, including, but not limited to, computer languages such as data-oriented languages (e.g., SQL, dBase), system languages (e.g., C, Objective-C, C++, Assembly), architectural languages (e.g., Java), and application languages (e.g., PHP, Ruby, Perl, Python). Instructions may also be implemented in computer languages such as array languages, aspectoriented languages, assembly languages, authoring languages, command line interface languages, compiled languages, concurrent languages, curly-bracket languages, dataflow languages, data-structured languages, declarative languages, esoteric languages, extension languages, fourth-generation languages, functional languages, interactive mode languages, interpreted languages, iterative languages, list-based languages, little languages, logic-based languages, machine languages, macro languages, metaprogramming languages, multiparadigm languages, numerical analysis, non-English-based languages, object-oriented class-based languages, object-oriented prototype-based languages, off-side rule languages, procedural languages, reflective languages, rule-based languages, scripting languages, stackbased languages, synchronous languages, syntax handling languages, visual languages, wirth languages, and xml-based languages. Memory 404 may also be used for storing temporary variable or other intermediate information during execution of instructions to be executed by processor 402. Computer system 400 further includes a data storage device 406 such as a magnetic disk or optical disk, coupled to bus 408 for storing information and instructions. Computer system 400 may be coupled via communications module 460 (e.g., communications module 114, 124, or 144 from FIG. 1) to various devices (not illustrated). The communications module 410 can be any input/output module. In certain embodiments not illustrated, the communications module 410 is configured to connect to a plurality of devices, such as an input device (e.g., input devices 118 or 152 from FIG. 1) and/or a display device (e.g., display devices 118 or 148 from FIG. 1).

According to one aspect of the present disclosure, the ADM 104, the console 120, or the server 140 can be implemented using a computer system 400 in response to processor 402 executing one or more sequences of one or more instructions contained in memory 404. Such instructions may be read into memory 404 from another machine-readable medium, such as data storage device 406. Execution of the sequences of instructions contained in main memory 404 causes processor 402 to perform the process steps described herein. One or more processors in a multi-processing arrangement may also be employed to execute the sequences of instructions contained in memory 404. In alternative embodiments, hard-wired circuitry may be used in place of or in combination with software instructions to implement various embodiments of the present disclosure. Thus, embodiments of the present disclosure are not limited to any specific combination of hardware circuitry and software.

The term "machine-readable medium" as used herein refers to any medium or media that participates in providing instructions to processor 402 for execution. Such a medium may take many forms, including, but not limited to, non-volatile media, volatile media, and transmission media. Non-volatile media include, for example, optical or magnetic disks, such as data storage device 406. Volatile media include dynamic memory, such as memory 404. Transmission media include coaxial cables, copper wire, and fiber optics, including the wires that comprise bus 408. Common forms of machine-readable media include, for example, floppy disk, a flexible disk, hard disk, magnetic tape, any other magnetic medium, a CD-ROM, DVD, any other optical medium, punch cards, paper tape, any other physical medium with patterns of holes, a RAM, a PROM, an EPROM, a FLASH EPROM, any other memory chip or cartridge, or any other medium from which a computer can read.

The disclosed systems and methods provide an alert management system in which alerts are displayed at medication dispensing stations, such as ADMs, that provide up to date information on clinical information specific to a patient and medication for the patient that may affect the dispensing of the specific medication to the specific patient. The alerts are provided from through a central console that is connected to a server that generates the alerts in response to requests by the medication dispensing stations.

All elements, parts and steps described herein are preferably included. It is to be understood that any of these elements, parts and steps may be replaced by other elements, parts and steps or deleted altogether as will be obvious to those skilled in the art.

The person skilled in the art will understand that the method steps mentioned in this description may be carried out by hardware including but not limited to processors; input devices comprising at least keyboards, mouse, scanners, cameras; output devices comprising at least monitors, printers. The method steps are to be carried out with the appropriate devices when needed. For example, a decision step could be carried out by a decisionmaking unit in a processor by implementing a decision algorithm. The person skilled in the art will understand that this decisionmaking unit can exist physically or effectively, for example in a computer's processor when carrying out the aforesaid decision algorithm. The above analysis is to be applied to other steps described herein.

## Claims

1. A system for providing alerts regarding dispense of potentially harmful medications to a patient comprising:
an input device (116) of an automated dispensing machine (104) and configured to receive, from a user, a first request to dispense a medication for a patient;
a communications module (114, 124) configured to provide, to a server (140) over a communication network (150) between the server (140) and the automated dispensing machine (104), a second request for information regarding the medication dispense for the patient, and to receive, from the server (140) in response to the second request, clinical information for the patient; and
a processor (112) configured to:
receive, via the input device (116), the first request to initiate a dispense of a medication from the automated dispensing machine (104), and a selection of the patient;
trigger, responsive to the first request and selection, the second request for information;
obtain, from the server (140) in response to the second request and the selection of the patient, the clinical information including critical information, **characterized in that** the critical information indicates
an out of range lab value, associated with the patient, that would be impacted such as to aggravate a patient condition if the medication is administered to the patient;
interrupt, responsive to the clinical information including the critical information and prior to dispensing the medication to the user, the dispense of the medication on the automated dispensing machine (104) to display on a display device (118), to the user, an alert that identifies the medication as potentially harmful to the patient based on the critical information, the user being prevented from interacting with software of the automated dispensing machine (104) until the alert is closed,
log off the user from the automated dispensing machine (104) when a timeout countdown expires without interaction between the user and the display device (118) that displays the alert, and
finish the dispense of the medication for the patient responsive to the user acknowledging the alert.

2. The system of Claim 1, wherein the critical information additionally comprises at least one of abnormal lab results or abnormal vital signs.

3. The system of Claim 1, wherein the clinical information is obtained after receipt of the request from the user.

4. The system of Claim 1, wherein the clinical information is updated based on a most recently recorded physician round.

5. The system of Claim 1, wherein the alert additionally comprises at least one of a time value, a medication, a query, an instruction, and an acknowledgement.

6. The system of Claim 1, wherein the alert additionally comprises a title of the alert, date of the alert, and a date of the last laboratory results associated with the alert.

7. A method for providing alerts regarding dispense of potentially harmful medications to a patient comprising:
receiving (202), from a user via an input device of an automated dispensing machine , a selection of a patient and a first request to dispense a medication for the patient;
providing (203), to a server, responsive to the first request and selection of the patient, a second request for information regarding the medication dispense for the patient;
receiving (208), from the server in response to the second request and the selection of the patient, clinical information that includes critical information **characterized in that** the critical information indicates an out of range lab value, associated with the patient, that would be impacted such as to aggravate a patient condition if the medication is administered to the patient;
interrupting, responsive to receiving the critical information and prior to dispensing the medication to the user, the dispense of the medication on the automated dispensing machine to display (210) an alert regarding the medication dispense, the alert including the indication that the medication is potentially harmful to the patient based on the critical information, the user being prevented from interacting with software of the automated dispensing machine until the alert is closed;
logging off the user from the automated dispensing machine when a timeout countdown expires without interaction between the user and the display device that displays the alert; and
finishing the medication dispense for the patient responsive to the user acknowledging the alert.

8. The method of Claim 7, wherein the critical information additionally comprises at least one of abnormal lab results or abnormal vital signs.

9. The method of Claim 7, wherein the clinical information is obtained after receipt of the request from the user.

10. The method of Claim 7, wherein the clinical information is updated based on a most recently recorded physician round.

11. The method of Claim 7, wherein the alert additionally comprises at least one of a time value, a medication, a query, an instruction, and an acknowledgement.

12. The method of Claim 7, wherein the alert additionally comprises a title of the alert, date of the alert, and a date of the last laboratory results associated with the alert.

13. A machine-readable medium comprising machine-readable instructions which, when executed on a data processing system, cause the data processing system to execute a method according to any of claims 7 to 12.

## Patentansprüche

1. System zum Bereitstellen von Warnmeldungen bezüglich der Abgabe von potenziell gefährlichen Medikamenten an einen Patienten, aufweisend:
eine Eingabevorrichtung (116) eines Ausgabeautomaten (104), die dafür ausgelegt ist, von einem Benutzer eine erste Anforderung zur Abgabe eines Medikaments für einen Patienten zu empfangen;
ein Kommunikationsmodul (114, 124), das dafür ausgelegt ist, einem Server (140) über ein Kommunikationsnetzwerk (150) zwischen dem Server (140) und dem Ausgabeautomaten (104) eine zweite Anforderung von Informationen bezüglich der Medikamentenabgabe für den Patienten bereitzustellen, und von dem Server (140) als Reaktion auf die zweite Anforderung klinische Informationen für den Patienten zu empfangen; und
einen Prozessor (112), der so ausgelegt ist, dass er:
über die Eingabevorrichtung (116) die erste Anforderung zum Einleiten einer Abgabe eines Medikaments aus dem Ausgabeautomaten (104) und eine Auswahl des Patienten empfängt;
als Antwort auf die erste Anforderung und die Auswahl die zweite Anforderung von Informationen auslöst;
vom Server (140) als Reaktion auf die zweite Anforderung und die Auswahl des Patienten die klinischen Informationen, darunter kritische Informationen, erhält, **dadurch gekennzeichnet, dass** die kritischen Informationen einen außerhalb eines Bereichs liegenden Laborwert angeben, der dem Patienten zugeordnet ist, der so beeinflusst würde, dass sich der Zustand des Patienten verschlimmert, wenn dem Patienten das Medikament verabreicht wird;
als Antwort auf die klinischen Informationen, die die kritischen Informationen enthalten, und vor der Abgabe des Medikaments an den Benutzer, die Abgabe des Medikaments am Ausgabeautomaten (104) unterbricht, um dem Benutzer auf einer Anzeigevorrichtung (118) eine Warnmeldung anzuzeigen, die beruhend auf den kritischen Informationen das Medikament als potentiell gefährlich für den Patienten erkennt, wobei der Benutzer daran gehindert ist, mit einer Software des Ausgabeautomaten (104) zu interagieren, bis die Warnmeldung geschlossen ist,
den Benutzer aus dem Ausgabeautomaten (104) ausloggt, wenn ein Zeitüberschreitungs-Countdown ohne Interaktion zwischen dem Benutzer und der Anzeigevorrichtung (118), die die Warnmeldung anzeigt, abläuft, und
die Abgabe des Medikaments für den Patienten als Antwort auf die Bestätigung der Warnmeldung durch den Benutzer beendet.

2. System nach Anspruch 1, wobei die kritischen Informationen zusätzlich abnormale Laborergebnisse und/oder abnormale Vitalparameter enthalten.

3. System nach Anspruch 1, wobei die klinischen Informationen nach Empfang der Anforderung vom Benutzer erhalten werden.

4. System nach Anspruch 1, wobei die klinischen Informationen beruhend auf einer zuletzt dokumentierten Arztvisite aktualisiert werden.

5. System nach Anspruch 1, wobei die Warnmeldung zusätzlich einen Zeitwert, ein Medikament, eine Abfrage, eine Anweisung und/oder eine Bestätigung umfasst.

6. System nach Anspruch 1, wobei die Warnmeldung zusätzlich eine Bezeichnung der Warnmeldung, ein Datum der Warnmeldung und ein Datum der letzten Laborergebnisse umfasst, die der Warnmeldung zugeordnet sind.

7. Verfahren zum Bereitstellen von Warnmeldungen bezüglich der Abgabe von potenziell gefährlichen Medikamenten an einen Patienten, umfassend:
Empfangen (202), von einem Benutzer über eine Eingabevorrichtung eines Ausgabeautomaten, einer Auswahl eines Patienten und einer ersten Anforderung zur Abgabe eines Medikaments für den Patienten;
Bereitstellen (203), an einen Server und als Antwort auf die erste Anforderung und die Auswahl des Patienten, einer zweiten Anforderung von Informationen bezüglich der Medikamentenabgabe für den Patienten;
Empfangen (208), vom Server als Reaktion auf die zweite Anforderung und die Auswahl des Patienten, von klinischen Informationen, die kritische Informationen enthalten, **dadurch gekennzeichnet, dass** die kritischen Informationen einen außerhalb eines Bereichs liegenden Laborwert angeben, der dem Patienten zugeordnet ist, der so beeinflusst würde, dass sich der Zustand des Patienten verschlimmert, wenn dem Patienten das Medikament verabreicht wird;
Unterbrechen, als Antwort auf den Empfang der kritischen Informationen und vor der Abgabe des Medikaments an den Benutzer, der Abgabe des Medikaments am Ausgabeautomaten, um eine Warnmeldung bezüglich der Medikamentenabgabe anzuzeigen (210), wobei die Warnmeldung die Angabe enthält, dass beruhend auf den kritischen Informationen das Medikament potentiell gefährlich für den Patienten ist, wobei der Benutzer daran gehindert ist, mit einer Software des Ausgabeautomaten zu interagieren, bis die Warnmeldung geschlossen ist;
Ausloggen des Benutzers aus dem Ausgabeautomaten, wenn ein Zeitüberschreitungs-Countdown ohne Interaktion zwischen dem Benutzer und der Anzeigevorrichtung, die die Warnmeldung anzeigt, abläuft; und
Beenden der Medikamentenabgabe für den Patienten als Antwort auf die Bestätigung der Warnmeldung durch den Benutzer.

8. Verfahren nach Anspruch 7, wobei die kritischen Informationen zusätzlich abnormale Laborergebnisse und/oder abnormale Vitalparameter enthalten.

9. Verfahren nach Anspruch 7, wobei die klinischen Informationen nach Empfang der Anforderung vom Benutzer erhalten werden.

10. Verfahren nach Anspruch 7, wobei die klinischen Informationen beruhend auf einer zuletzt dokumentierten Arztvisite aktualisiert werden.

11. Verfahren nach Anspruch 7, wobei die Warnmeldung zusätzlich einen Zeitwert, ein Medikament, eine Abfrage, eine Anweisung und/oder eine Bestätigung umfasst.

12. Verfahren nach Anspruch 7, wobei die Warnmeldung zusätzlich eine Bezeichnung der Warnmeldung, ein Datum der Warnmeldung und ein Datum der letzten Laborergebnisse umfasst, die der Warnmeldung zugeordnet sind.

13. Maschinenlesbares Medium, das maschinenlesbare Anweisungen enthält, die bei Ausführung auf einem Datenverarbeitungssystem bewirken, dass das Datenverarbeitungssystem ein Verfahren nach einem der Ansprüche 7 bis 12 ausführt.

## Revendications

1. Système de notification d'alertes relatives à la distribution de médicaments potentiellement nocifs à un patient comprenant :
un dispositif d'entrée (116) d'une machine de distribution automatisée (104) configuré pour recevoir, depuis un utilisateur, une première demande de distribuer un médicament pour un patient ;
un module de communication (114, 124) configuré pour fournir, à un serveur (140) via un réseau de communication (150) entre le serveur (140) et la machine de distribution automatisée (104), une deuxième demande d'informations relatives à la distribution de médicament pour le patient, et pour recevoir, depuis le serveur (140) en réponse à la deuxième demande, des informations cliniques pour le patient ; et
un processeur (112) configuré pour :
recevoir, via le dispositif d'entrée (116), la première demande d'initier une distribution d'un médicament depuis la machine de distribution automatisée (104), et une sélection du patient ;
déclencher, en réponse à la première demande et à la sélection, la deuxième demande d'informations ;
obtenir, depuis le serveur (140) en réponse à la deuxième demande et à la sélection du patient, les informations cliniques incluant des informations critiques, **caractérisées en ce que** les informations critiques indiquent une valeur de laboratoire hors tolérance, associée au patient, qui serait impactée de manière à aggraver un état du patient si le médicament est administré au patient ;
interrompre, en réponse aux informations cliniques incluant les informations critiques et avant de distribuer le médicament à l'utilisateur, la distribution du médicament sur la machine de distribution automatisée (104) pour afficher sur un dispositif d'affichage (118), à l'utilisateur, une alerte qui identifie le médicament comme potentiellement nocif pour le patient sur la base des informations critiques, l'utilisateur étant empêché d'interagir avec un logiciel de la machine de distribution automatisée (104) jusqu'à ce que l'alerte soit fermée,
déconnecter l'utilisateur de la machine de distribution automatisée (104) lorsqu'un délai d'attente expire sans interaction entre l'utilisateur et le dispositif d'affichage (118) qui affiche l'alerte, et
terminer la distribution du médicament pour le patient en réponse au fait que l'utilisateur accuse réception de l'alerte.

2. Le système de la revendication 1, sachant que les informations critiques comprennent en outre au moins un terme parmi des résultats de laboratoire anormaux ou des signes vitaux anormaux.

3. Le système de la revendication 1, sachant que les informations cliniques sont obtenues après réception de la demande depuis l'utilisateur.

4. Le système de la revendication 1, sachant que les informations cliniques sont mises à jour sur la base d'une tournée d'un médecin enregistrée le plus récemment.

5. Le système de la revendication 1, sachant que l'alerte comprend en outre au moins un terme parmi une valeur de temps, un médicament, une requête, une instruction, et un accusé de réception.

6. Le système de la revendication 1, sachant que l'alerte comprend en outre un titre de l'alerte, une date de l'alerte, et une date des derniers résultats de laboratoire associés à l'alerte.

7. Procédé de notification d'alertes relatives à la distribution de médicaments potentiellement nocifs à un patient comprenant :
la réception (202), depuis un utilisateur via un dispositif d'entrée d'une machine de distribution automatisée, d'une sélection d'un patient et d'une première demande de distribuer un médicament pour le patient ;
la fourniture (203), à un serveur, en réponse à la première demande et à la sélection du patient, d'une deuxième demande d'informations relatives à la distribution de médicament pour le patient ;
la réception (208), depuis le serveur en réponse à la deuxième demande et à la sélection du patient, d'informations cliniques qui incluent des informations critiques, **caractérisées en ce que** les informations critiques indiquent une valeur de laboratoire hors tolérance, associée au patient, qui serait impactée de manière à aggraver un état du patient si le médicament est administré au patient ;
l'interruption, en réponse à la réception des informations critiques et avant de distribuer le médicament à l'utilisateur, de la distribution du médicament sur la machine de distribution automatisée pour afficher (210) une alerte relative à la distribution de médicament, l'alerte incluant l'indication que le médicament est potentiellement nocif pour le patient sur la base des informations critiques, l'utilisateur étant empêché d'interagir avec un logiciel de la machine de distribution automatisée jusqu'à ce que l'alerte soit fermée ;
la déconnexion de l'utilisateur de la machine de distribution automatisée lorsqu'un délai d'attente expire sans interaction entre l'utilisateur et le dispositif d'affichage qui affiche l'alerte ; et
le fait de terminer la distribution de médicament pour le patient en réponse au fait que l'utilisateur accuse réception de l'alerte.

8. Le procédé de la revendication 7, sachant que les informations critiques comprennent en outre au moins un terme parmi des résultats de laboratoire anormaux ou des signes vitaux anormaux.

9. Le procédé de la revendication 7, sachant que les informations cliniques sont obtenues après réception de la demande depuis l'utilisateur.

10. Le procédé de la revendication 7, sachant que les informations cliniques sont mises à jour sur la base d'une tournée d'un médecin enregistrée le plus récemment.

11. Le procédé de la revendication 7, sachant que l'alerte comprend en outre au moins un terme parmi une valeur de temps, un médicament, une requête, une instruction, et un accusé de réception.

12. Le procédé de la revendication 7, sachant que l'alerte comprend en outre un titre de l'alerte, une date de l'alerte, et une date des derniers résultats de laboratoire associés à l'alerte.

13. Support lisible par machine comprenant des instructions lisibles par machine qui, lorsqu'elles sont exécutées sur un système de traitement de données, font exécuter au système de traitement de données un procédé selon l'une quelconque des revendications 7 à 12.
